# EUROPEAN PATENT SPECIFICATION

(11) **EP 2 319 520 B1**
(45) Date of publication and mention of the grant of the patent: **20.03.2013**
(21) Application number: 09766499.9
(22) Date of filing: 16.05.2009
(51) Int. Cl.: A61K 36/48, A23L 1/30, A61P 37/02, A61P 43/00

(54) **IMMUNOSTIMULATING AGENT**
IMMUNSTIMULIERENDES MITTEL
AGENT IMMUNOSTIMULANT

(30) Priority: 19.06.2008 JP 2008161015
(43) Date of publication of application: 11.05.2011
(73) Proprietor: National University Corporation Hokkaido University, Sapporo-shi Hokkaido 060-0808 (JP); Nepuree Corporation, Tokyo 104-0031 (JP)
(72) Inventor: KANO, Tsutomu, Tokyo 104-0031 (JP); NISHIMURA, Takashi, Sapporo-shi Hokkaido 060-0815 (JP)
(74) Representative: Wasner, Marita
(86) International application number: PCT/JP2009/059097
(87) International publication number: WO 2009/154051

(56) References cited:
- WO-A1-2007/020755
- JP-A- 9 176 033
- JP-A- 2005 118 015
- JP-A- 2006 296 251
- JP-A- 2007 106 718
- TANAKA S ET AL: "The extract of Japanese soybean, Kurosengoku activates the production of IL-12 and IFN-gamma by DC or NK1.1<+> cells in a TLR4- and TLR2-dependent manner", CELLULAR IMMUNOLOGY, ACADEMIC PRESS, SAN DIEGO, CA, US, vol. 266, no. 2, 1 January 2011 (2011-01-01), pages 135-142, XP027554664, ISSN: 0008-8749 [retrieved on 2010-10-01]
- CHAN-HO OH ET AL.: 'Effect of small Black Soybean Fraction on the T cell-mediated Immune Responses in vivo and Proliferation of Leukemia Cells in vitro' NATURAL PRODUCT SCIENCES vol. 13, no. 2, 2007, pages 123 - 127, XP008142507
- HOKAIDO SHINBUN CHOKAN CHIHO KUCHI 10 November 2007, page 28, XP008144514
- NIPPON NOGYO SHINBUN KIJI JOHO 04 January 2005, page 5, XP008144513
- HOKKAIDO SHINBUN CHOKAN CHIHO KUCHI 18 January 2008, page 29, XP008144512
- THE NIKKAN KOGYO SHINBUN, LTD. no. 213, 02 April 2008, page 30, XP008144511

## Description

### Technical Field

The present invention relates to an immunostimulant comprising Kurosengoku (*Glycine max* cv. Kurosengoku) beans, crushed Kurosengoku beans, or an aqueous ethanol solution extract thereof, particularly, Kurosengoku beans swollen with water, crushed Kurosengoku beans, or an aqueous ethanol solution extract thereof, as an active ingredient. The present application is a patent application subject to the Article 19 of the Industrial Technology Enhancement Act, the Project Sponsored by the Regional Science and Technology Promotion Project, Ministry of Education, Culture, Sports, Science and Technology, 2008.

### Background Art

The immune system is especially important among many factors constituting the host defense mechanism and plays an important role in defense against infections with microbes (e.g., bacteria, yeast, fungi, and viruses) or tumors or the like and in the maintenance of homeostasis. Its main mechanism is based on a foreign matter clearance ability enhanced by activating antigen-presenting cells (e.g., dendritic cells and macrophages) which recognize microbes or tumors as foreign matter, or so-called acquired immune cells (e.g., T lymphocytes and B lymphocytes) stimulated from the antigen-presenting cells, either in an antigen-specific manner or in an antigen-nonspecific manner via receptors recognizing bacterial components or lectin or the like.

Particularly, the antigen-presenting cells in the body incorporate therein foreign matter by phagocytosis and digest this invader for clearance, while they have the function of presenting, to lymphocytes, peptides of the digested foreign matter expressed on the cell surface. In addition, these antigen-presenting cells are known to deeply participate in the activation of the immune system, in such a way that they participate in the activation of other immune cells such as T cells or B cells by the acquirement of toxicity to cancer cells or the release of various cytokines including interleukin-12 (IL-12). Therefore, the activation of the antigen-presenting cells is an important challenge to the activation of immunity.

On the other hand, the functions of the immune system are known to be reduced due to some factor such as aging, stress, fatigue, or environmental factors. Since these factors are aspects that frequently occur in daily life, it seems preferable to constantly or routinely activate immunity by means of diet or supplement ingestion or the like, rather than to activate immunity every time the functions are reduced.

Various foods or supplements or a large number of immunostimulants containing Chinese medicine formulations or natural extracts (e.g., herbal medicines) have been studied and developed for the purpose of such constant or routine activation of immunity. Examples thereof can include kefir grains (Patent Literature 1), Isomaltooligosaccharide (Patent Literature 2), *Porphyra* extracts (Patent Literature 3), soybean protein (Patent Literature 4), and water-soluble lignin (Patent Literature 5).

Kurosengoku (*Glycine max* cv. Kurosengoku) beans is one variety of black soybean whose cultivation was discontinued around the 1970's due to great care required for the cultivation. Recently, black soybean has been reevaluated owing to its nutritional values (rich in soybean isoflavone and anthocyanin) and favorable tastes, and the cultivation of Kurosengoku has thus been resumed. However, the use thereof is restricted to the range of general food materials such as Natto (fermented soybean), black soybean tea, millet products, and sushi roll.
Citation List
Patent Literature

Patent Literature 1: Japanese Patent Laid-Open No. 2-65790
Patent Literature 2: Japanese Patent Laid-Open No. 2-174718
Patent Literature 3: Japanese Patent Laid-Open No. 3-284626
Patent Literature 4: Japanese Patent Laid-Open No. 4-229189
Patent Literature 5: Japanese Patent Laid-Open No. 2-237934

### Summary of Invention

### Technical Problem

An object of the present invention is to provide a novel natural material having an immune balance-modulating effect, particularly, an immunostimulating ability.

### Solution to Problem

The present inventors have completed each aspect of the invention below by finding that Kurosengoku (*Glycine max* cv. Kurosengoku) beans, crushed Kurosengoku beans, or an aqueous ethanol solution extract thereof, particularly, Kurosengoku beans swollen with water, crushed Kurosengoku beans, or an aqueous ethanol solution extract thereof, exhibits an immunostimulating ability that is not observed in other varieties of black soybean, and is capable of suppressing the increase of polarized Th2 immunity.

(1) An immune balance modulator comprising Kurosengoku (*Glycine max* cv. Kurosengoku) beans, crushed Kurosengoku beans, or an aqueous ethanol solution extract thereof as an active ingredient.

(2) The immune balance modulator according to (1), wherein the Kurosengoku beans are Kurosengoku beans swollen with water.

(3) The immune balance modulator according to (2), wherein the Kurosengoku beans swollen with water are Kurosengoku beans boiled in water or Kurosengoku beans steamed with water vapor.

(4) The immune balance modulator according to (1), wherein the crushed Kurosengoku beans are a crushed product of water-swollen Kurosengoku beans or a water-swollen product of crushed Kurosengoku beans.

(5) The immune balance modulator according to (4), wherein the crushed product of water-swollen Kurosengoku beans or the water-swollen product of crushed Kurosengoku beans are a crushed product of water-boiled Kurosengoku beans or a water-boiled product of crushed Kurosengoku beans, or a crushed product of water vapor-steamed Kurosengoku beans or a water vapor-steamed product of crushed Kurosengoku beans.

(6) The immune balance modulator according to (4) or (5), wherein the crushed Kurosengoku beans are crushed Kurosengoku beans that are collected into a supernatant fraction at 400xg and into a precipitate fraction at 18000xg in centrifugation with water as a mobile phase.

(7) The immune balance modulator according to any of (2) to (6), wherein the Kurosengoku beans or the crushed Kurosengoku beans are further dehydrated.

(8) The immune balance modulator according to any of (1) to (7), wherein the aqueous ethanol solution is a 30 vol% aqueous ethanol solution and/or a 60 vol% aqueous ethanol solution.

(9) The immune balance modulator according to any of (1) to (7), wherein the aqueous ethanol solution extract is an extract that is collected into a supernatant fraction at 485xg in centrifugation with an aqueous ethanol solution as a mobile phase.

(10) The immune balance modulator according to any of (1) to (9), wherein the immune balance modulator is intended for activation of an antigen-presenting cell.

(11) The immune balance modulator according to any of (1) to (9), wherein the immune balance modulator is intended for activation of a splenic immune cell.

(12) Use of Kurosengoku (*Glycine max* cv. Kurosengoku) beans, crushed Kurosengoku beans, or an aqueous ethanol solution extract thereof as an immune balance-modulating food.

(13) The use according to (12), wherein the Kurosengoku beans are Kurosengoku beans swollen with water.

(14) The use according to (13), wherein the Kurosengoku beans swollen with water are Kurosengoku beans boiled in water or Kurosengoku beans steamed with water vapor.

(15) The use according to (12), wherein the crushed Kurosengoku beans are a crushed product of water-swollen Kurosengoku beans or a water-swollen product of crushed Kurosengoku beans.

(16) The use according to (15), wherein the crushed product of water-swollen Kurosengoku beans or the water-swollen product of crushed Kurosengoku beans are a crushed product of water-boiled Kurosengoku beans or a water-boiled product of crushed Kurosengoku beans, or a crushed product of water vapor-steamed Kurosengoku beans or a water vapor-steamed product of crushed Kurosengoku beans.

(17) The use according to (15) or (16), wherein the crushed Kurosengoku beans are crushed Kurosengoku beans that are collected into a supernatant fraction at 400xg and into a precipitate fraction at 18000xg in centrifugation with water as a mobile phase.

(18) The use according to any of (13) to (17), wherein the Kurosengoku beans or the crushed Kurosengoku beans are further dehydrated.

(19) The use according to any of (12) to (18), wherein the food is intended for activation of an antigen-presenting cell.

(20) The use according to any of (12) to (18), wherein the food is intended for activation of a splenic immune cell.

(21) An immune balance modulator comprising crushed Kurosengoku beans as an active ingredient, the crushed Kurosengoku beans being obtained by centrifuging a ground product of water-swollen Kurosengoku beans (*Glycine max* cv. Kurosengoku) at 400xg with water as a mobile phase and further centrifuging the collected supernatant fraction at 18000xg to collect the crushed product as a precipitate fraction.

(22) An immune balance modulator comprising an extract as an active ingredient, the extract being obtained by centrifuging a ground product of water-swollen Kurosengoku beans (*Glycine max* cv. Kurosengoku) at 800xg with water as a mobile phase, adding a 30 vol% aqueous ethanol solution to the collected precipitate fraction, conducting filtration treatment, and centrifuging the collected filtrate at 485xg to collect the extract into a supernatant fraction.

(23) An immune balance modulator comprising an extract as an active ingredient, the extract being obtained by centrifuging a ground product of water-swollen Kurosengoku beans (*Glycine max* cv. Kurosengoku) at 800xg with water as a mobile phase, adding a 30 vol% aqueous ethanol solution to the collected precipitate fraction, conducting filtration treatment, further adding a 60 vol% aqueous ethanol solution to the collected solid matter, conducting filtration treatment, and centrifuging the collected filtrate at 485xg to collect the extract into a supernatant fraction.

### Advantageous Effects of Invention

An immune balance modulator of the present invention is a safe immune balance modulator that utilizes natural material Kurosengoku beans, and can activate immune functions by activating immune cells important for host defense, particularly, antigen-presenting cells. At the same time, the immune balance modulator of the present invention can also maintain the balance between Th1 immunity and Th2 immunity by suppressing polarized Th2 immunity and can achieve the enhancement of immune-mediated host defense functions and the suppression of undesirable allergic reaction.

### Brief Description of Drawings

[Figure 1] Figure 1 is a graph showing a capability of inducing IFNγ production shown in Example 1 as to Kurosengoku beans or crushed Kurosengoku beans.
[Figure 2] Figure 2 is graph showing a capability of inducing IFNγ production as to a crushed product prepared from Kurosengoku beans differing in harvest year.
[Figure 3] Figure 3 is a graph showing comparison test results of a capability of inducing IFNγ production as to a soluble fraction and a precipitate fraction (crushed product) prepared from Kurosengoku and other beans.
[Figure 4] Figure 4 is a graph showing the growth-promoting effect of a crushed product prepared from Kurosengoku beans differing in harvest year, on splenic immune cells.
[Figure 5] Figure 5 shows flow cytometry analysis results showing the rate of activated cells increased by the coculture of NK1.1-positive NK and NKT cells, CD11c-positive dendritic cells, CD19-positive B cells, or TCRβ-positive T cells with crushed Kurosengoku beans.
[Figure 6] Figure 6 shows flow cytometry analysis results showing that coculture with crushed Kurosengoku beans significantly promotes the expression of MHC class II molecules and CD86 molecules on DC surface.
[Figure 7] Figure 7 shows flow cytometry analysis results showing that coculture with crushed Kurosengoku beans significantly promotes the expression of MHC class I molecules, MHC class II molecules, CD40, and CD86 on DC surface.
[Figure 8] Figure 8 is a graph showing comparison test results of a capability of promoting IL-12p70 production from DC as to a precipitate fraction (crushed product) prepared from Kurosengoku and other beans.
[Figure 9] Figure 9 is a graph showing a capability of inducing IFNγ production as to crushed Kurosengoku beans dried using ethanol.
[Figure 10] Figure 10 is a graph showing a capability of inducing IFNγ production as to a crushed product from Kurosengoku beans flour.
[Figure 11] Figure 11 is a graph showing that crushed Kurosengoku beans are recognized by TLR2 or TLR4 to induce IFNγ production.
[Figure 12] Figure 12 is a graph showing the IFNγ production-inducing effects of the water-soluble fraction, 30 vol% ethanol-extracted fraction, 60 vol% ethanol-extracted fraction, and 100% ethanol-extracted fraction of Kurosengoku beans on splenic immune cells.
[Figure 13] Figure 13 is a diagram showing that the 60 vol% ethanol-extracted fraction is recognized by TLR2 and TLR4 to induce IFNγ production from splenic immune cells.
[Figure 14] Figure 14 is a diagram showing that the 60 vol% ethanol-extracted fraction is recognized by TLR2 and TLR4 to induce IL-12p70 production from bone marrow-derived dendritic cells.
[Figure 15] Figure 15 is a diagram showing that the 60 vol% ethanol-extracted fraction more induces IFNγ production from human peripheral blood mononuclear cells.

### Description of Embodiments

The present invention provides an immunostimulant comprising Kurosengoku (*Glycine max* cv. Kurosengoku) beans, crushed Kurosengoku beans, or an aqueous ethanol solution extract thereof as an active ingredient. Kurosengoku, one variety of black soybean (scientific name: *Glycine max*), is a unique variety differing in many properties from other varieties of black soybean in such a way that: its fruit is spherical in shape; it is much smaller (weight of 100 beans: approximately 10 to 11 g) than those of other varieties of black soybean; its soybean isoflavone and anthocyanin contents are higher than those of other varieties of black soybean; and usual soybean has 9 to 10 leaves, whereas Kurosengoku has 13 to 14 leaves.

Kurosengoku beans are produced and supplied in, for example, Hokuryu-cho, Uryu-gun, Hokkaido, Japan or Otobecho, Nishi-gun, Hokkaido, Japan with the Hokkaido Kurosengoku Productive Cooperation (31-1, Hekisui, Hokuryu-cho, Uryu-gun, Hokkaido, Japan) as the driving force. Moreover, Kurosengoku beans are also produced and supplied in Shizukuishi-cho, Iwate-gun, Iwate, Japan. In the present invention, any of these soybeans of Kurosengoku can be used, regardless of the production origins. The present invention adds a new functional value to Kurosengoku beans or processed products of Kurosengoku beans such as Natto (fermented soybean).

In one aspect of the immune balance modulator of the present invention, Kurosengoku beans, particularly, Kurosengoku beans swollen with water, is directly used. The Kurosengoku beans swollen with water may be prepared by dipping in an appropriate amount (preferably, an excessive amount) of water all night and all day, boiling during dipping in an excessive amount of water, or steaming with water vapor, to the extent that the Kurosengoku beans are ground or crushed using a mortar and a pestle. Moreover, in the present invention, the Kurosengoku beans thus swollen with water can be dried by lyophilization or the like for use. In the present invention, the phrase "Kurosengoku beans swollen with water" is meant to encompass Kurosengoku beans swollen with water and dried products thereof, unless otherwise specified. A preferable aspect of the immune balance modulator of the present invention is "Kurosengoku beans swollen with water" that are obtained by boiling Kurosengoku beans after addition of water in an amount of approximately 10 times by weight that of the Kurosengoku beans.

An alternative preferable aspect of the immune balance modulator of the present invention is a Kurosengoku-bean-derived crushed product. Examples of the Kurosengoku-bean-derived crushed product can include: crushed Kurosengoku beans that are obtained by grinding or crushing raw or heat-roasted Kurosengoku beans without swelling with water at any timing; crushed Kurosengoku beans that are obtained by water-swelling a crushed product obtained by grinding or crushing raw or heat-roasted Kurosengoku beans; crushed Kurosengoku beans that are obtained by grinding or crushing water-swollen Kurosengoku beans; and crushed Kurosengoku beans that are obtained by grinding or crushing a dried product of water-swollen Kurosengoku beans. Specifically, the immune balance modulator of the present invention may comprise any crushed Kurosengoku beans, regardless of whether the Kurosengoku beans before being crushed are raw, heat-roasted, water-swollen, or dried after water-swelling and regardless of the order of crushing and water swelling operations. Moreover, the crushed Kurosengoku beans or crushed product also encompass powder.

Furthermore, the crushed Kurosengoku beans that are obtained by water-swelling a crushed product obtained by grinding or crushing raw or heat-roasted Kurosengoku beans, and the crushed Kurosengoku beans that are obtained by grinding or crushing water-swollen Kurosengoku beans may be dehydrated by drying treatment such as drying by heating, drying in air, or lyophilization and then used. Specifically, in the present invention, both water-containing crushed Kurosengoku beans and a dehydrated product thereof can be used.

In the present invention, the phrase "crushed product of water-swollen Kurosengoku beans" is meant to encompass both of the water-containing crushed Kurosengoku beans and the dehydrated product thereof. Moreover, the phrase "crushed Kurosengoku beans" is meant to encompass all the aspects of Kurosengoku-bean-derived crushed products according to the present invention, including crushed Kurosengoku beans without swelling with water at any timing and the "crushed product of water-swollen Kurosengoku beans".

The operation of grinding or crushing Kurosengoku beans or "Kurosengoku beans swollen with water" can be performed using a mortar and a pestle, a mixer, a blender, or other general tools, apparatuses, or methods capable of crushing beans and does not particularly require special tools, apparatuses, methods, etc.

A first preferable example of the "crushed Kurosengoku beans" that may be used in the present invention is crushed Kurosengoku beans that are obtained by grinding or crushing raw or heat-roasted Kurosengoku beans. This crushed Kurosengoku beans may be used directly as an immune balance modulator or may be used as an immune balance modulator after being prepared as the "crushed product of water-swollen Kurosengoku beans" by water-swelling the crushed Kurosengoku beans by dipping in an excessive amount of water. As described above, a dehydrated product of this crushed Kurosengoku beans may be used as the "crushed product of water-swollen Kurosengoku beans".

A second preferable example of the "crushed Kurosengoku beans" that may be used in the present invention is a "crushed product of water-swollen Kurosengoku beans" that is obtained by grinding Kurosengoku beans boiled with an excessive amount of water, together with the broth, using a mortar and a pestle and then collecting the crushed product on a filter by filtration treatment. This crushed Kurosengoku beans are colored almost white, and most of plant pigments such as anthocyanin and polyphenol, which are the sources of the dark brown color of Kurosengoku beans, are presumably moved to a filtrate fraction in the filtration treatment. As described above, a dehydrated product of this crushed Kurosengoku beans may be used as the "crushed product of water-swollen Kurosengoku beans".

Moreover, a third preferable example of the "crushed Kurosengoku beans" that may be used in the present invention is a "crushed product of water-swollen Kurosengoku beans" that is finer than that of the first example and is obtained by grinding Kurosengoku beans boiled with an excessive amount of water, together with the broth, using a mortar and a pestle, then collecting a supernatant fraction by centrifugation at 400xg, and further centrifuging this supernatant fraction at 18000xg to collect the crushed product as a precipitate fraction. The crushed Kurosengoku beans are also colored almost white, as in the first example. As described above, a dehydrated product of the crushed Kurosengoku beans may be used as the "crushed product of water-swollen Kurosengoku beans".

In another preferable embodiment of the immune balance modulator of the present invention, an aqueous ethanol solution extract of the Kurosengoku beans, particularly, the Kurosengoku beans swollen with water, or an aqueous ethanol solution extract of the crushed Kurosengoku beans, particularly, the crushed product of water-swollen Kurosengoku beans, is used. The ethanol concentration of the aqueous ethanol solution used in the extraction is not particularly limited and can be selected appropriately. The ethanol concentration is preferably set to 30 vol%, more preferably 60 vol%.

A first preferable example of the "aqueous ethanol solution extract" that may be used in the present invention is an extract that is obtained by grinding Kurosengoku beans boiled with an excessive amount of water, together with the broth, using a mortar and a pestle, then collecting a precipitate fraction by centrifugation treatment, to which an excessive amount of an aqueous ethanol solution is then added for filtration treatment to collect a filtrate, and subjecting this filtrate to centrifugation treatment to collect the extract as a supernatant. The amount of the aqueous ethanol solution added is not particularly limited as long as it is an excessive amount. The amount can be set to, for example, a 10-fold amount. Moreover, each supernatant fraction may be used directly as the "aqueous ethanol solution extract" or can be used after being collected and then appropriately subjected to lyophilization or the like.

A second preferable example of the "aqueous ethanol solution extract" that may be used in the present invention is an extract that is obtained by grinding Kurosengoku beans boiled with an excessive amount of water, together with the broth, using a mortar and a pestle, then collecting a precipitate fraction by centrifugation treatment, to which an excessive amount of an aqueous ethanol solution is then added for filtration treatment, further adding an excessive amount of an aqueous ethanol solution to the collected solid matter on the filter for filtration treatment to collect a filtrate, and subjecting this filtrate to centrifugation treatment to collect the extract as a supernatant. In this case, the step of further adding an excessive amount of an aqueous ethanol solution to the obtained solid matter, conducting filtration treatment to collect a filtrate may be performed once or twice or more, and the concentration of the excessive amount of the aqueous ethanol solution added can be selected appropriately. For example, the extract can be obtained by grinding Kurosengoku beans boiled with an excessive amount of water, together with the broth, using a mortar and a pestle, then collecting a precipitate fraction by centrifugation treatment, to which an excessive amount of a 30 vol% aqueous ethanol solution is then added for filtration treatment, adding an excessive amount of a 60 vol% aqueous ethanol solution to the collected solid matter on the filter for filtration treatment to collect a filtrate, and further subjecting this filtrate to centrifugation treatment to collect the extract as a supernatant. The amount of the aqueous ethanol solution added is not particularly limited as long as it is excessive. The amount can be set to, for example, a 10-fold amount. Moreover, the supernatant fraction may be used directly as the "aqueous ethanol solution extract" or can be used after being collected and then appropriately subjected to lyophilization or the like.

Moreover, a third preferable example of the "aqueous ethanol solution extract" that may be used in the present invention is an extract that can be obtained by grinding Kurosengoku beans boiled with an excessive amount of water, together with the broth, using a mortar and a pestle, then collecting a precipitate fraction by centrifugation at 800xg, to which an excessive amount of a 30 vol% aqueous ethanol solution is then added for filtration treatment, adding an excessive amount of a 60 vol% aqueous ethanol solution to the collected solid matter on the filter for filtration treatment to collect a filtrate, and further subjecting this filtrate to centrifugation treatment at 485xg to collect the extract as a supernatant. The amount of the aqueous ethanol solution added is not particularly limited as long as it is excessive. The amount can be set to, for example, a 10-fold amount. Moreover, the supernatant fraction may be used directly as the "aqueous ethanol solution extract" or can be used after being collected and then appropriately subjected to lyophilization or the like.

In the present invention, the Kurosengoku, "Kurosengoku beans swollen with water", "crushed Kurosengoku beans", or "aqueous ethanol solution extract" thereof, particularly, the "Kurosengoku beans swollen with water", "crushed product of water-swollen Kurosengoku beans", or "aqueous ethanol solution extract" thereof, is used as an immune balance modulator, as an immunostimulant, as an immune balance-modulating drink or food, or as an immunostimulating drink or food. Specifically, the present invention relates to use of the Kurosengoku, "Kurosengoku beans swollen with water", "crushed Kurosengoku beans", or "aqueous ethanol solution extract" thereof, particularly, the "Kurosengoku beans swollen with water", "crushed product of water-swollen Kurosengoku beans", or "aqueous ethanol solution extract" thereof, as an immune balance modulator or an immune balance-modulating drink or food, particularly, as an immunostimulant or an immunostimulating drink or food. In other words, the present invention relates to use of the Kurosengoku, "Kurosengoku beans swollen with water", "crushed Kurosengoku beans", or "aqueous ethanol solution extract" thereof, particularly, the "Kurosengoku beans swollen with water", "crushed product of water-swollen Kurosengoku beans", or "aqueous ethanol solution extract" thereof, for the purpose of or with the aim of modulating immune balance in an animal or enhancing, potentiating, or activating its immunity. In the present invention, the Kurosengoku, "Kurosengoku beans swollen with water", "crushed Kurosengoku beans", or "aqueous ethanol solution extract" thereof is collectively referred to as the "Kurosengoku beans, crushed Kurosengoku beans, or aqueous ethanol solution extract thereof".

In the present invention, the modulation of immune balance, i.e., the immune balance-modulating effect, means an effect of bringing Th1 immunity and Th2 immunity to a modulated state by activating one of these immunities, particularly, Th1 immunity, and attenuating the increase of polarized Th2 immunity. In the present invention, the modulation of immune balance is used exchangeably with the control of immune balance. Moreover, the "immunostimulation" means the enhancement, potentiation, or activation of immunity, particularly, Th1 immunity, which is a host defense mechanism possessed by animals, particularly, mammals including humans, against foreign matter or malignant neoplasms or the like.

In general, the Th1 immunity is understood as cellular immunity involving Th1 cells (T helper 1 cells) induced by the presentation of antigenic peptides from dendritic cells or macrophages (antigen-presenting cells) and by the action of IL-12. The Th1 cells produce IL-2, TNF-α, and the like in addition to cytokines (e.g., IFNγ) that suppress IgE antibody production via the inhibition of Th2 cell differentiation or the inhibition of B cell maturation. They also function to activate killer T cells or to enhance the activity of the antigen-presenting cells such as dendritic cells or macrophages. On the other hand, the Th2 immune system is understood as humoral immunity involving Th2 cells (T helper 2 cells) induced by the presentation of antigenic peptides from macrophages (antigen-presenting cells) and by the action of IL-4. The Th2 cells produce IL-5, IL-6, and IL-10 in addition to cytokines (e.g., IL-4 and IL-13) that increase IgE antibody production mediated by B cell maturation.

IL-4 and IL-10 produced from the Th2 cells are known to mutually modulate their actions to suppress IFNγ production from Th1 cells. Moreover, when the Th2 immunity is dominant, suppressed cellular immunity is thought to result in susceptibility to allergy due to easily increased severity of infectious diseases and increased IgE antibody production mediated by B cell maturation. Thus, the collapse of balance between Th1 immunity and Th2 immunity, particularly, the increase or dominancy of functions of the polarized Th2 immune system, is not always favorable to the living body.

The "Kurosengoku beans, crushed Kurosengoku beans, or aqueous ethanol solution extract thereof" used in the present invention have, as shown in Examples below, an immunostimulating effect to activate immune cells such as antigen-presenting cells, NK cells, T cells, and B cells, i.e., to activate Th1 immunity and enhance, potentiate, or activate immunity. Moreover, the "Kurosengoku beans, crushed Kurosengoku beans, or aqueous ethanol solution extract thereof" used in the present invention exhibit the effect of inducing IFNγ production important for the inhibition of Th2 cell differentiation or the inhibition of B cell maturation and the effect of inducing the production of IL-12, a cytokine important for the induction of IFNγ production from NK cells or T cells. Furthermore, through such an immunostimulating effect, IFNγ production-inducing effect, or IL-12 production-inducing effect, the "Kurosengoku beans, crushed Kurosengoku beans, or aqueous ethanol solution extract thereof" can alleviate a state with dominancy of functions of the Th2 immune system, for example, allergy, by attenuating polarized Th2 immunity.

In this way, the "Kurosengoku beans, crushed Kurosengoku beans, or aqueous ethanol solution extract thereof" can be used as an immune balance modulator via its immunostimulating ability. Specifically, the immune balance modulator of the present invention can be expected to have a preventive or therapeutic effect on allergic diseases such as atopic dermatitis, skin roughness, sensitive skin, pollinosis, asthma, bronchial asthma, rhinitis, and urticaria, or an improving effect on their symptoms.

Moreover, the "Kurosengoku beans, crushed Kurosengoku beans, or aqueous ethanol solution extract thereof" used in the present invention have a Th1 immunity-enhancing, potentiating, or activating effect, i.e., an immunostimulating effect, as described above, and can be used as an immunostimulant. Furthermore, the "Kurosengoku beans, crushed Kurosengoku beans, or aqueous ethanol solution extract thereof" used in the present invention can also be used as an IFNγ production-inducing agent or as an IL-12 production-inducing agent. Thus, the immune balance modulator and the immunostimulant comprising the "Kurosengoku beans, crushed Kurosengoku beans, or aqueous ethanol solution extract thereof" as an active ingredient can be expected to have a preventive or therapeutic effect on infectious diseases caused by viruses, bacteria, and the like or malignant neoplasms or the like, or an improving effect on their symptoms. Particularly, the continuous ingestion of the "Kurosengoku beans, crushed Kurosengoku beans, or aqueous ethanol solution extract thereof" used in the present invention are expected to be able to improve constitution to insusceptibility to infections with viruses, bacteria, and the like or insusceptibility to development of malignant neoplasms.

Furthermore, the "Kurosengoku beans, crushed Kurosengoku beans, or aqueous ethanol solution extract thereof" used in the present invention directly act on antigen-presenting cells. Owing to this action, the "Kurosengoku beans, crushed Kurosengoku beans, or aqueous ethanol solution extract thereof" have the effect of increasing the expression of major histocompatibility complex (MHC) class I molecules and MHC class II molecules that play an important role in antigen presentation to T cells, and further has the effect of increasing the expression of CD86 and CD40 molecules that play an auxiliary role in the activation of T cells and the like. Thus, the "Kurosengoku beans, crushed Kurosengoku beans, or aqueous ethanol solution extract thereof" according to the present invention are also useful as an activator for antigen-presenting cells.

The "Kurosengoku beans, crushed Kurosengoku beans, or aqueous ethanol solution extract thereof" used in the present invention can be used directly as an immune balance modulator, particularly, as an immunostimulant. In addition, the "Kurosengoku beans, crushed Kurosengoku beans, or aqueous ethanol solution extract thereof" may be used after being formulated in the forms of emulsions, oil solutions, wettable powders, powders, tablets, capsules, solutions, or other general dosage forms by preparing compositions in combination with appropriate excipients, emulsifiers, dispersants, suspending agents, spreading agents, penetrants, humectants, stabilizers, and the like. Orally ingestible dosage forms are particularly preferable.

The amount of the "Kurosengoku beans, crushed Kurosengoku beans, or aqueous ethanol solution extract thereof" formulated in these compositions or dosage forms is not particularly limited and is preferably set to 1 to 99 wt%, more preferably 10 to 99 wt%, even more preferably 50 to 99 wt%, in terms of dry solid content in the whole amount of the composition or formulation.

These compositions or various dosage forms may be prepared in the forms of pharmaceutical or quasi drugs formulated, if necessary, with drugs (e.g., vitamins or herbal medicines) other than the "Kurosengoku beans, crushed Kurosengoku beans, or aqueous ethanol solution extract thereof", as an active ingredient. Alternatively, the "Kurosengoku beans, crushed Kurosengoku beans, or aqueous ethanol solution extract thereof" can also be used after being processed into drink or food or supplement forms having an immune balance-modulating effect or immunostimulating effect by combination with food materials containing starch, protein, mineral components, or other nutrients, or with food additives.

Examples of the drink or food forms include, but not limited to: confectionary such as chewing gums, candies, tablet-type confectionary, gumdrops, biscuits, and snacks; frozen desserts such as ice creams, sherbets, and ices; Japanese confectionary such as Yokan (adzuki bean jelly), Monaka (adzuki bean jam filling sandwiched between wafers), and Amanatto (sweet fermented beans); and puddings and jams. The amount of the "Kurosengoku beans, crushed Kurosengoku beans, or aqueous ethanol solution extract thereof" added to these drinks or foods differs depending on their forms and is preferably set to 0.001 wt% or more and to 5 wt% or less in terms of taste. The "Kurosengoku beans, crushed Kurosengoku beans, or aqueous ethanol solution extract thereof" in such a drink or food form are understood as one embodiment of the present invention as long as it is used for the purpose of or with the aim of modulating immune balance or enhancing, potentiating, or activating immunity.

Hereinafter, the present invention will be described more specifically with reference to Examples. However, the present invention is not limited to these Examples in its interpretation.

### Examples

### <Example 1> Capability of inducing IFNγ production by Kurosengoku beans swollen with water

### (1) Preparation of Kurosengoku beans swollen with water

Approximately 20 g of Kurosengoku beans were weighed. Then, the surface was repetitively washed with milliQ water to thoroughly remove the dirt on the surface. After further addition of 200 mL of milliQ water, the Kurosengoku beans were swollen with water under pressure at 121°C for 20 minutes in an autoclave and collected using a paper filter. This Kurosengoku beans swollen with water were further frozen at -30°C and then dried using a lyophilizer.

### (2) Preparation of crushed product of water-swollen Kurosengoku beans

Approximately 10 g of Kurosengoku beans were weighed. The surface was repetitively washed with milliQ water to thoroughly remove the dirt on the surface. After further addition of 60 mL of milliQ water, the Kurosengoku beans were swollen with water under pressure at 121°C for 20 minutes in an autoclave.

The Kurosengoku beans were separated from the broth using a paper filter, transferred to a mortar, and ground using a pestle while the broth was added thereto. The ground Kurosengoku beans were transferred together with the broth to a 50-mL tube and subjected to centrifugation treatment at 1500 rpm (485xg) for 10 minutes to collect a supernatant fraction. 10 mL of this supernatant fraction was transferred to a 15-mL tube and subjected again to centrifugation treatment at 1500 rpm (485xg) for 10 minutes to collect a supernatant fraction.

1 mL of this supernatant fraction after the second centrifugation treatment was transferred to a 1.5-mL Eppendorf tube and subjected to centrifugation treatment at 15,000 rpm (17360xg) for 10 minutes to respectively obtain a soluble fraction and a crushed product of water-swollen Kurosengoku beans as a precipitate fraction.

### (3) Separation of splenic immune cells

The spleen was collected from 7-week-old C57BL/6 female mice purchased from Charles River Laboratories Japan Inc. The spleen was dissociated using tweezers in an RPMI-1640 medium (Wako Pure Chemical Industries, Ltd.) containing 10% FCS, 2.38 mg/mL Hepes, 0.11 mg/mL sodium pyruvate, 200 U/mL penicillin G, and 0.1 mg/mL streptomycin. The cells were collected together with the culture solution through a nylon mesh (Wako Pure Chemical Industries, Ltd.) while the tissue portions were removed. After centrifugation treatment at 1500 rpm for 5 minutes using a small refrigerated centrifuge (himac CF7D2, HITACHIKOKI Co., Ltd.), the supernatant was discarded, and red blood cells were eliminated by incubation at 37°C for 90 seconds with 2 mL of 0.155 M ammonium chloride to prepare splenic immune cells in the RPMI-1640 medium.

### (4) Confirmation of IFNγ production-inducing effect

The lyophilized sample obtained in the paragraph (1) was suspended in PBS and then added at a final concentration of 0.06 mg/mL to 4 mg/mL to the splenic immune cells in the RPMI-1640 medium of (3), followed by coculture. Moreover, the soluble fraction obtained in (2) was added at a final dilution ratio of 5- to 320-fold to the splenic immune cells in the RPMI-1640 medium of (3), while the crushed Kurosengoku beans as the precipitate fraction was added at a final dilution ratio of 250- to 16000-fold thereto, followed by coculture at 37°C in a 5% CO₂ atmosphere. The coculture was performed using 96-well flat bottom plates (Nunc) with a splenic immune cell concentration set to 5×10⁵ cells/well. 48 hours later, the amount of IFNγ in each culture supernatant was quantified using ELISA Mouse IFNγ BD OptEIA set (BD Biosciences).

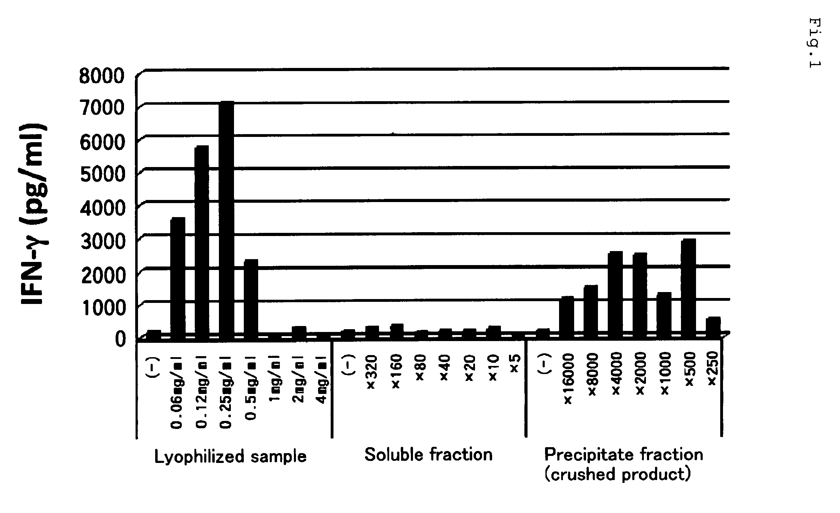

As a result, a capability of inducing IFNγ production from splenic immune cells was confirmed in the lyophilized sample of (1) and the crushed Kurosengoku beans of (2). By contrast, no significant capability of inducing IFNγ production from splenic immune cells was confirmed in the soluble fraction of (2) (Figure 1).

### <Example 2> Influence of harvest year of Kurosengoku beans on IFNγ production-inducing effect

Crushed Kurosengoku beans (precipitate fraction) were prepared by the same operation as in Example 1 (2) from Kurosengoku beans (harvested in the year 2005, 2006, or 2007 and stored at room temperature) and examined for its capability of inducing IFNγ production from splenic immune cells by the same operation as in Example 1 (4) except for dilution ratios.

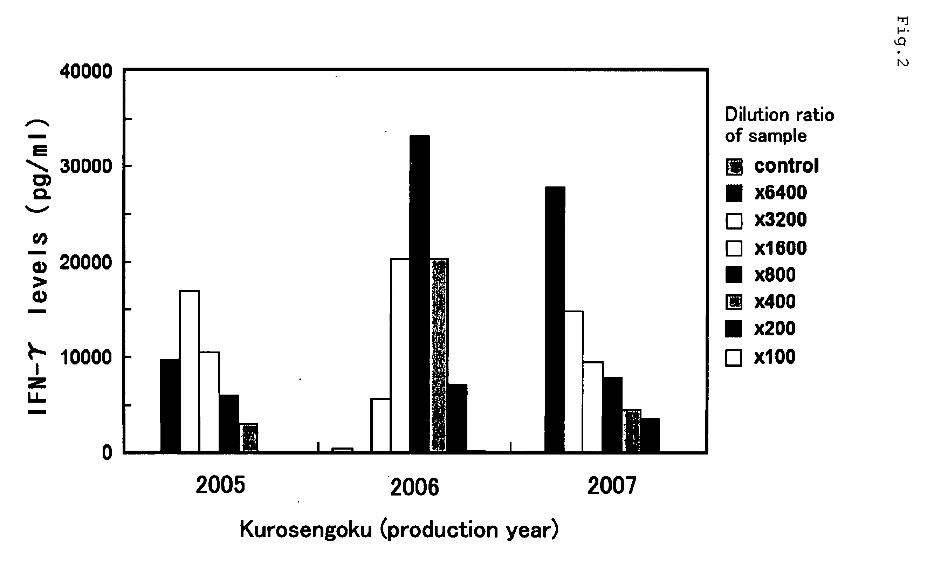

As a result, all the crushed Kurosengoku bean samples were confirmed to have a capability of inducing IFNγ production from splenic immune cells, regardless of the harvest years (Figure 2).

### <Example 3> Comparison of IFNγ production-inducing effect between Kurosengoku beans and other beans

A soluble fraction and a precipitate fraction were prepared from each of *Vigna unguiculata* (Cowpea) of Chinese origin, *Vigna angularis* (Adzuki bean) of Hokkaido (Japan) origin (*Vigna angularis-1,* trade name "Tokachi San Otsubu Teyori Mame"), *Vigna angularis* of Hokkaido origin (*Vigna angularis-2), Glycine max* (yellow soybean) of Miyagi (Japan) origin (trade name "Tsuru No Ko Daizu"), *Phaseolus angularis* cv. Dainagon of Hokkaido origin, and *Glycine max* (black soybean) of Hokkaido origin by the same operation as in Example 1 (2) and examined for their capabilities of inducing IFNγ production from splenic immune cells by the same operation as in Example 1 (4) except for dilution ratios.

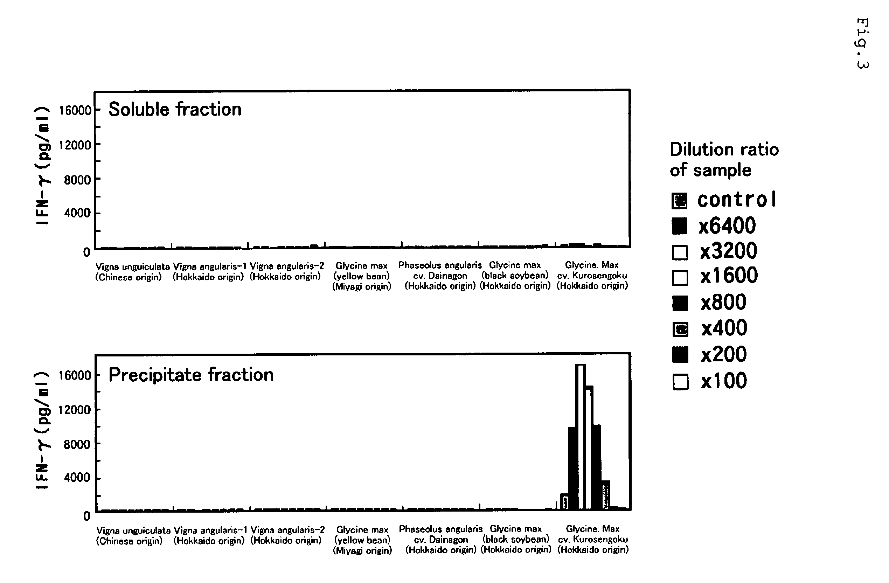

As a result, it was demonstrated that none of the soluble and precipitate fractions prepared from the beans except for the Kurosengoku beans have a significant capability of inducing IFNγ production from splenic immune cells (Figure 3).

### <Example 4> Confirmation of growth-promoting effect of Kurosengoku beans on splenic immune cell

Crushed Kurosengoku beans prepared from each Kurosengoku beans differing in harvest year in Example 2 were cocultured with splenic immune cells for 48 hours. After the completion of the coculture, a portion of the culture supernatant was removed from each well, and an appropriate amount of TetraColor ONE reagent (SEIKAGAKU CORP.) was added instead, followed by incubation for additional 2 hours. After the incubation, the absorbance of each well at 450 nm and 630 nm was measured using a microplate reader (EZA-ABS, IWAKI GLASS CO., LTD.). From the value of difference between these absorbances (absorbance at 450 nm - absorbance at 630 nm), the degree of cell growth can be quantified.

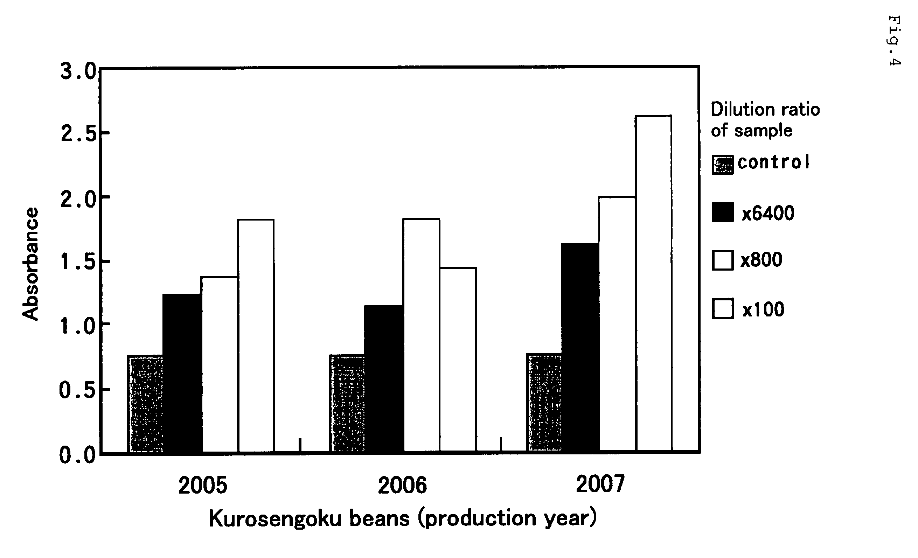

As a result, it was demonstrated that the addition of the crushed Kurosengoku beans to the medium promotes splenic immune cell growth, compared with the control non-supplemented with the crushed Kurosengoku beans (Figure 4).

### <Example 5>

For the splenic immune cells cocultured with crushed Kurosengoku beans (precipitate fraction of Example 1 (2) diluted 1000-fold) of Example 1 (4), NK1.1-positive NK and NKT cells, CD11c-positive dendritic cells, CD19-positive B cells, or TCRβ-positive T cells in the culture solution after 36-hour culture were stained with an anti-NK1.1+ (PK136), anti-CD11c+ (HL3), anti-CD19+ (1D3), or anti-TCRβ+ (H57-597) monoclonal antibody and detected using flow cytometry (FACS Calibur, BD Biosciences). Each of these molecules is a typical surface antigen on splenic immune cells. Also, CD69 molecules, which are early activation antigens on cell surface antigen-positive cells of the cells described above, were stained with an anti-CD69 monoclonal antibody (H1.2F3). A group involving a non-supplemented medium was prepared as a non-supplemented control.

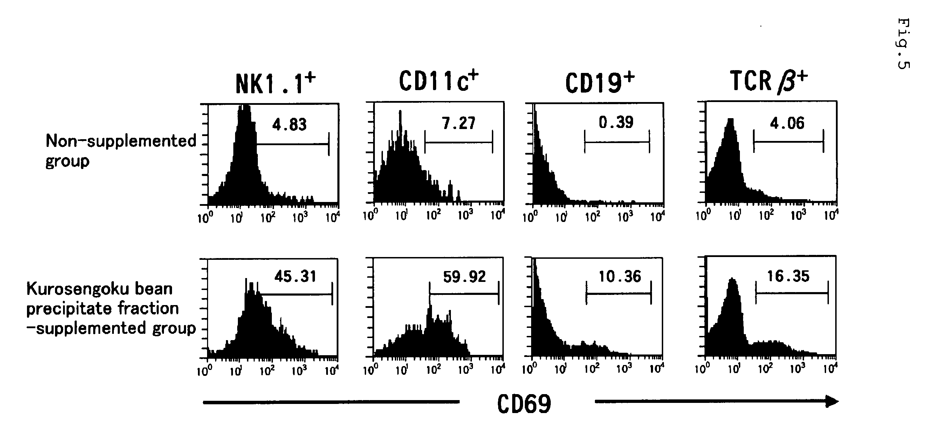

As a result, it was demonstrated that coculture with the crushed Kurosengoku beans significantly increases the number of cells having the cell surface antigens, compared with the non-supplemented control (Figure 5).

### <Example 6>

Bone marrow cells were collected from the thigh bones of 7-week-old C57BL/6 female mice purchased from Charles River Laboratories Japan Inc., inoculated at a concentration of 1×10⁶ cells/well to 6-well flat bottom plates (Nunc), and cultured for 6 days in the presence of 10 ng/mL GM-CSF (PeproTech Inc.) to induce dendritic cells (DC; antigen-presenting cells). The DC cells were cocultured with the crushed Kurosengoku beans of Example 1 (2) (precipitate fraction, diluted 1000-fold) in an RPMI-1640 medium containing 10% FCS, 2.38 mg/mL Hepes, 0.11 mg/mL sodium pyruvate, 200 U/mL penicillin G, and 0.1 mg/mL streptomycin. The expression levels of MHC class II molecules and CD86 molecules on the cell surface after 36 hours were detected by flow cytometry (FACS Calibur, BD Biosciences) using an anti-MHC class II molecule monoclonal antibody (AF6-88.5) and an anti-CD86 monoclonal antibody (GL1).

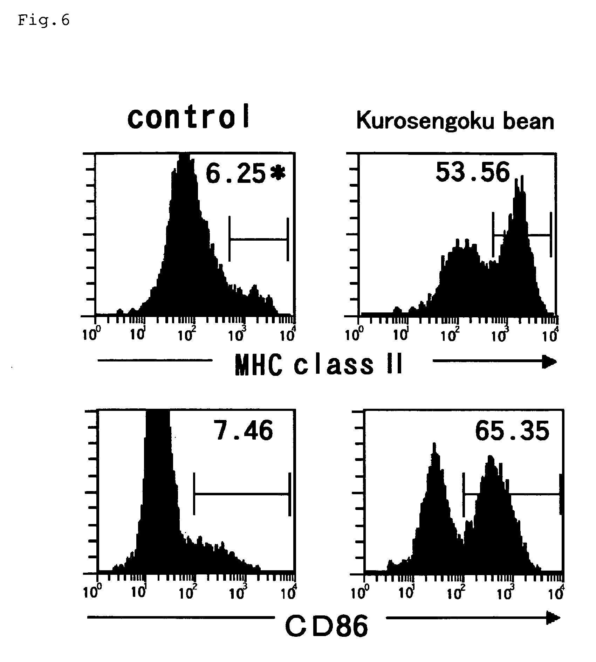

As a result, it was demonstrated that the addition of the crushed Kurosengoku beans significantly promotes the expression of MHC class II molecules and CD86 molecules, compared with the crushed Kurosengoku-bean-non-supplemented control (Figure 6).

### <Example 7>

The DC cells prepared in Example 6 were cocultured with the crushed Kurosengoku beans of Example 1 (2) (precipitate fraction, diluted 1000-fold) in an RPMI-1640 medium containing 10% FCS, 2.38 mg/mL Hepes, 0.11 mg/mL sodium pyruvate, 200 U/mL penicillin G, and 0.1 mg/mL streptomycin. The expression levels of MHC class I molecules, MHC class II molecules, CD40, and CD86 on the DC surface after 0, 4, and 8 hours were detected by flow cytometry (FACS Calibur, BD Biosciences) using an anti-MHC class I molecule monoclonal antibody (AF6-120.1), an anti-MHC class II molecule monoclonal antibody (AF6-88.5), an anti-CD40 monoclonal antibody (3/23), and an anti-CD86 monoclonal antibody (GL1).

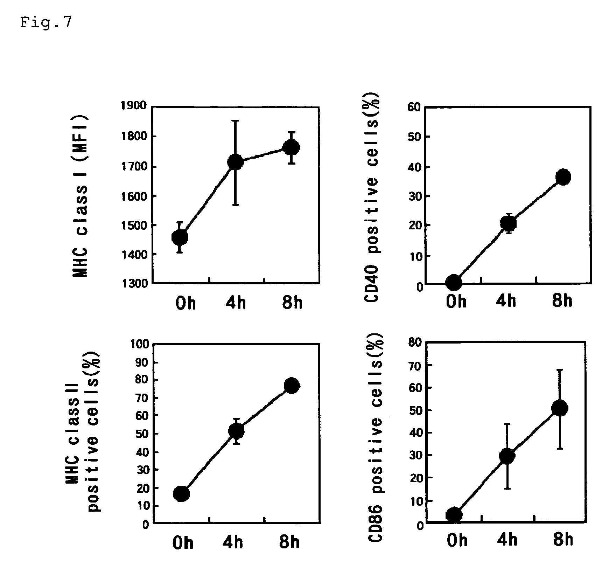

As a result, it was demonstrated that the addition of the crushed Kurosengoku beans significantly promotes the expression of MHC class I molecules, MHC class II molecules, CD40, and CD86 on the DC surface, compared with the crushed Kurosengoku-bean-non-supplemented control (Figure 7).

### <Example 8> Comparison of antigen-presenting cell-activating effect between Kurosengoku beans and other beans

The DC cells prepared in Example 6 were cocultured with each of the various bean-derived crushed products (precipitate fraction) prepared in Example 3 for 16 hours in the RPMI-1640 medium to prepare culture supernatants. IL-12p70 in these media was quantified using Mouse IL-12BD OptEIA set (BD Biosciences).

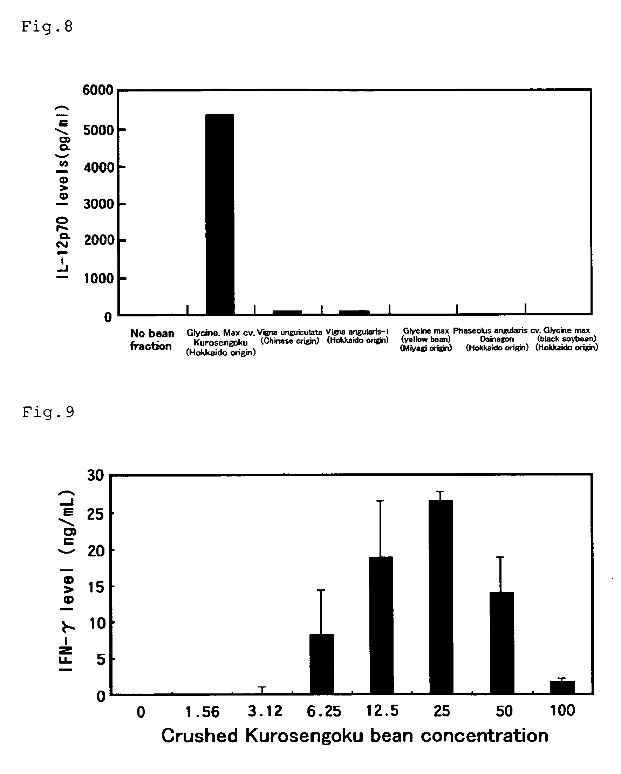

As a result, the Kurosengoku-bean-derived crushed product had a significant capability of promoting IL-12p70 production from DC, whereas no capability of promoting IL-12p70 production from DC was confirmed in the other bean-derived crushed products (Figure 8).

### <Example 9>

The precipitate fraction (crushed product) prepared in Example 1 (2) was suspended in 100% ethanol, then spread in plastic Petri dishes, and dried overnight in a desiccator shaded from the light. The residual solid matter was collected and used as a sample. This sample was adjusted to a final concentration of 0 to 100 µg/mL and evaluated for its capability of inducing IFNγ production from mouse splenic immune cells in the same way as in Example 1 (4).

As a result, a capability of inducing IFNγ production was also confirmed in the crushed Kurosengoku beans dried with 100% ethanol (Figure 9).

### <Example 10>

### (1) Preparation of Kurosengoku beans flour

Dry Kurosengoku beans with a 15% or less water content were heat-roasted with uniform stirring at 100°C to 150°C for 15 minutes to 20 minutes in a roasting pot treated with far-infrared radiation, and then completely roasted by heat-roasting for 30 seconds to 1 minute at a temperature further increased to 180°C to 200°C. 500 mg of Kurosengoku beans flour obtained from this heat-roasted Kurosengoku beans was suspended in 2 mL of milliQ water, thoroughly stirred, and then centrifuged at 1500 rpm (485xg). Its supernatant fraction was collected and centrifuged again at 15000 rpm (17360xg). The collected precipitate fraction was suspended in 100 µL of PBS to obtain crushed Kurosengoku beans. The crushed Kurosengoku beans were used in evaluation for its capability of inducing IFNγ production from mouse splenic immune cells in the same way as in Example 1 (4).

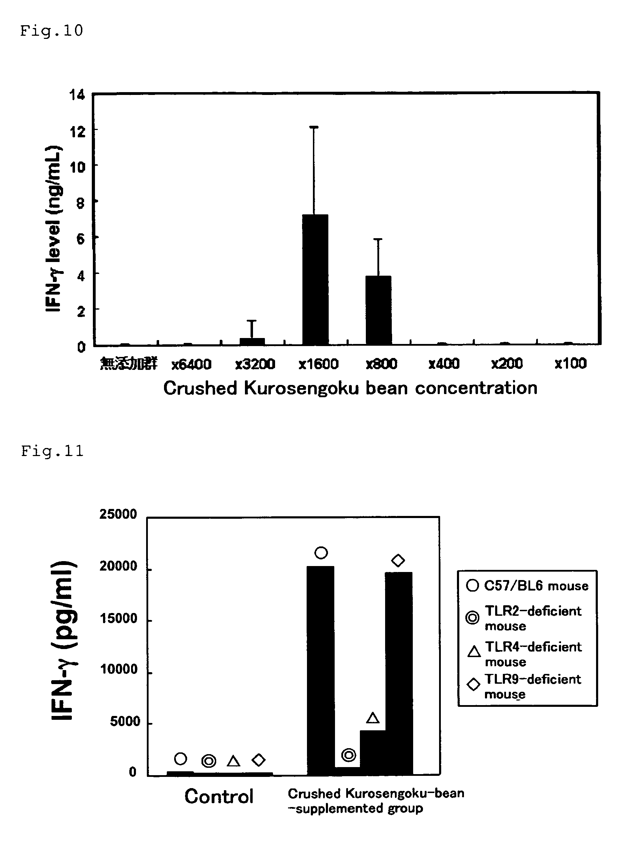

As a result, a capability of inducing IFNγ production was also confirmed in the crushed product from Kurosengoku beans flour (Figure 10).

### <Example 11> Study on TLR dependency of IFNγ production-inducing effect of Kurosengoku beans

The spleen was collected from each of 7-week-old C57BL/6 female mice purchased from Charles River Laboratories Japan Inc., TLR2 (Toll Like Receptor 2)-deficient mice, TLR4 (Toll Like Receptor 4)-deficient mice, and TLR9 (Toll Like Receptor 9)-deficient mice. Splenic immune cells in the RPMI-1640 medium were prepared by the same operation as in Example 1 (3). A crushed product of water-swollen Kurosengoku beans was obtained as a precipitate fraction by the same operation as in Example 1 (2) and added at a 200-fold dilution ratio to the splenic immune cells in the RPMI-1640 medium, followed by coculture at 37°C in 5% CO₂ atmosphere. The coculture was performed using 96-well flat bottom plates (Nunc) with a splenic immune cell concentration set to 5x105 cells/well. 48 hours later, the amount of IFNγ in each culture supernatant was quantified using ELISA Mouse IFNγ BD OptEIA set (BD Biosciences). Moreover, the amount of IFNγ in each culture supernatant from splenic immune cells cultured in the same way as above without adding the crushed product of water-swollen Kurosengoku beans (precipitate fraction) was quantified and used as a control.

As a result, high IFNγ production, compared with the control, was confirmed in the culture supernatants of the splenic immune cells of the C57BL/6 female mice and TLR9-deficient mice cocultured with the crushed Kurosengoku beans added. By contrast, IFNγ production was observed, compared with the control, in the culture supernatants of the splenic immune cells of the TLR2-deficient mice and TLR4-deficient mice cocultured with the crushed Kurosengoku beans added. However, this IFNγ production was confirmed to be significantly attenuated, compared with that in the culture supernatants of the splenic immune cells of the C57BL/6 female mice and TLR9-deficient mice. This demonstrated that the crushed product of water-swollen Kurosengoku beans (precipitate fraction) is recognized by TLR2 or TLR4 to induce IFNγ production (Figure 11).

### <Example 12> Confirmation of IFNγ production-inducing effect of ethanol-extracted fraction of Kurosengoku beans

Approximately 10 g of Kurosengoku beans were weighed. Then, the surface was repetitively washed with milliQ water to thoroughly remove the dirt on the surface. After further addition of 60 mL of milliQ water, the Kurosengoku beans were swollen with water under pressure at 121°C for 20 minutes in an autoclave.

The Kurosengoku beans were separated from the broth using a paper filter, transferred to a mortar, and ground using a pestle while the broth was added thereto, to prepare a crushed Kurosengoku bean suspension. This suspension was subjected to centrifugation treatment at 3000 rpm (800xg) for 20 minutes to obtain a precipitate fraction and a supernatant fraction. This supernatant fraction was dried using a lyophilizer to prepare a water-soluble fraction.

The precipitate fraction was suspended in a 10-fold amount of a 30 vol% aqueous ethanol solution and stirred for 30 minutes. Then, 30 vol% ethanol-extracted solid matter was separated from a 30 vol% ethanol-extracted filtrate using a filter paper (Whatman plc.). The 30 vol% ethanol-extracted filtrate was subjected to centrifugation treatment at 1500 rpm (485xg) for 15 minutes. The obtained supernatant fraction was treated with a concentration centrifuge (EYELA) for ethanol evaporation and then cooled in liquid nitrogen. The solvent was completely removed in a lyophilizer to prepare a 30 vol% ethanol-extracted fraction.

Subsequently, the 30 vol% ethanol-extracted solid matter was suspended in a 10-fold amount of a 60 vol% aqueous ethanol solution and stirred for 30 minutes. Then, 60 vol% ethanol-extracted solid matter was separated from a 60 vol% ethanol-extracted filtrate using a filter paper (Whatman plc.). The 60 vol% ethanol-extracted filtrate was treated in the same way as in the 30 vol% ethanol-extracted filtrate to prepare a 60 vol% ethanol-extracted fraction.

Furthermore, the 60 vol% ethanol-extracted solid matter was suspended in a 10-fold amount of a 100% aqueous ethanol solution and stirred for 30 minutes. Then, 100% ethanol-extracted solid matter was separated from a 100% ethanol-extracted filtrate using a filter paper (Whatman plc.). The 100% ethanol-extracted filtrate was treated in the same way as in the 30 vol% and 60 vol% ethanol-extracted filtrates to prepare a 100% ethanol-extracted fraction.

Each of the water-soluble fraction, 30 vol% ethanol-extracted fraction, 60 vol% ethanol-extracted fraction, and 100% ethanol-extracted fraction thus prepared was suspended in PBS and then added at a final concentration of 6.25 µg/mL to 400 µg/mL to splenic immune cells in the RPMI-1640 medium obtained by the same operation as in Example 1 (3). The amount of IFNγ was quantified in the same way as in Example 1 (4).

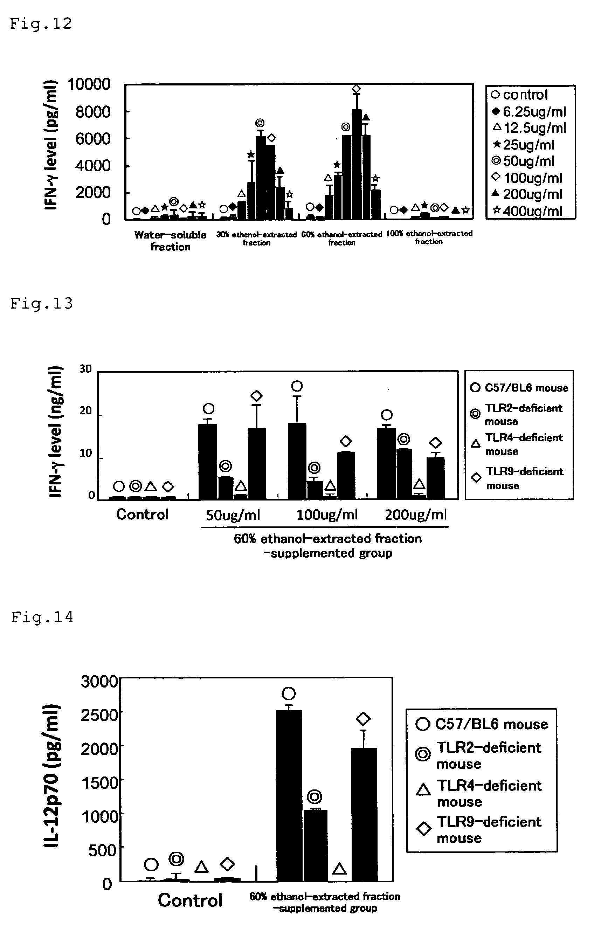

As a result, little IFNγ production was confirmed in all the water-soluble fraction- and 100% ethanol-extracted fraction-supplemented samples. By contrast, significant IFNγ production was confirmed in all the samples supplemented with the 12.5 µg/mL to 400 µg/mL 30 vol% ethanol-extracted fractions or 60 vol% ethanol-extracted fractions. Particularly, very significant IFNγ production was confirmed in the samples supplemented with the 50 µg/mL to 100 µg/mL 30 vol% ethanol-extracted fractions and the 50 µg/mL to 200 µg/mL 60 vol% ethanol-extracted fractions (Figure 12).

### <Example 13> Study on TLR dependency of IFNγ production-inducing effect of 60 vol% ethanol-extracted fraction

A 60 vol% ethanol-extracted fraction prepared by the same operation as in Example 12 was suspended in PBS and then added at a final concentration of 50 µg/mL, 100 µg/mL, or 200 µg/mL to splenic immune cells in the RPMI-1640 medium obtained by the same operation as in Example 1 (3). The amount of IFNγ was quantified in the same way as in Example 1 (4).

As a result, high IFNγ production, compared with the control, was confirmed in the culture supernatants of the splenic immune cells of the C57BL/6 female mice and TLR9-deficient mice cocultured with the 60 vol% ethanol-extracted fraction added at any of the concentrations. Moreover, IFNγ production was observed, compared with the control, in the culture supernatant of the splenic immune cells of the TLR2-deficient mice cocultured with the 60 vol% ethanol-extracted fraction added. However, this IFNγ production was confirmed to be significantly attenuated in the samples supplemented with the 50 µg/mL or 100 µg/mL 60 vol% ethanol-extracted fraction, compared with that in the culture supernatants of the splenic immune cells of the C57BL/6 female mice and TLR9-deficient mice supplemented with the same concentration of the 60 vol% ethanol-extracted fraction. Furthermore, little IFNγ production was confirmed in the culture supernatant of the splenic immune cells of the TLR4-deficient mice cocultured with the 60 vol% ethanol-extracted fraction added at any of the concentrations. These results demonstrated that the 60 vol% ethanol-extracted fraction is recognized by TLR2 or TLR4, particularly, by TRL4, to induce IFNγ production (Figure 13).

### <Example 14> Study on TLR dependency of IL-12p70 production-inducing effect of 60 vol% ethanol-extracted fraction

Bone marrow cells were collected from the thigh bones of 7-week-old C57BL/6 female mice purchased from Charles River Laboratories Japan Inc., TLR2-deficient mice, TLR4-deficient mice, or TLR9-deficient mice. DC was induced by the same operation as in Example 6. A 60 vol% ethanol-extracted fraction was obtained by the same operation as in Example 12, suspended in PBS, and then cocultured at a final concentration of 200 µg/mL with each DC by the same operation as in Example 8. 12 hours later, IL-12p70 in each culture supernatant was quantified by the same operation as in Example 8. Moreover, the amount of IL-12p70 in each culture supernatant of DC cultured in the same way as above without adding the 60 vol% ethanol-extracted fraction was quantified and used as a control.

As a result, high IL-12p70 production, compared with the control, was confirmed in the culture supernatants of DC of the C57BL/6 female mice and TLR9-deficient mice cocultured with the 60 vol% ethanol-extracted fraction added. Moreover, IL-12p70 production was observed, compared with the control, in the culture supernatant of DC of the TLR2-deficient mice cocultured with the 60 vol% ethanol-extracted fraction added. However, this IFNγ production was confirmed to be significantly attenuated, compared with that in the culture supernatants of DC of the C57BL/6 female mice and TLR9-deficient mice. Furthermore, no IL-12p70 production was confirmed, as in the control, in the culture supernatant of DC of the TLR4-deficient mice. These results demonstrated that the 60 vol% ethanol-extracted fraction is recognized by TLR2 or TLR4, particularly, by TLR4, to induce IL-12p70 production (Figure 14).

### <Example 15> Study on IFNγ production-inducing effect of 60 vol% ethanol-extracted fraction on human peripheral blood mononuclear cells

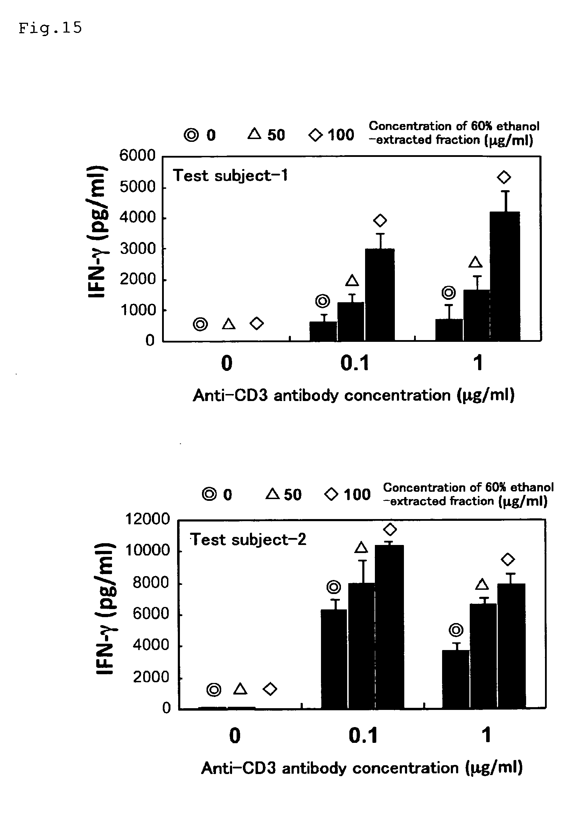

Peripheral blood was collected from two healthy test subjects (in Figure 15, indicated in test subject-1 and test subject-2). Peripheral blood mononuclear cells were isolated using a gravity separation solution for lymphocytes (Sigma-Aldrich Corp.), and peripheral blood mononuclear cells in the RPMI-1640 medium were prepared for each of the test subjects. This solution was inoculated at a peripheral blood mononuclear cell concentration of 2×10⁵ cells/well to 96-well flat bottom plates (Nunc). T cells were stimulated by the addition of an anti-CD3 antibody (Miltenyi Biotec) at a concentration of 0.1 µg/mL or 1 µg/mL to each well. Then, a 60 vol% ethanol-extracted fraction prepared by the same operation as in Example 12 was suspended in PBS and then added at a final concentration of 50 µg/mL or 100 µg/mL to the peripheral blood mononuclear cells in the RPMI-1640 medium, followed by coculture at 37°C in a 5% CO₂ atmosphere. 48 hours later, the amount of IFNγ in each culture supernatant was quantified using human IFNγ BD OptEIA set (BD Biosciences). Moreover, the amount of IFNγ in each culture supernatant of peripheral blood mononuclear cells cultured in the same way as above without adding the anti-CD3 antibody and/or the 60 vol% ethanol-extracted fraction was quantified and used as a control.

As a result, little IFNγ production was confirmed in the culture supernatant of the anti-CD3-antibody-non-supplemented peripheral blood mononuclear cells of any of the test subjects. By contrast, IFNγ production, compared with the control, was confirmed in the samples supplemented with the 0.1 µg/mL or 1 µg/mL anti-CD3 antibody. Moreover, this amount of IFNγ produced was increased by the addition of the 60 vol% ethanol-extracted fraction, and the addition of the 100 µg/mL 60 vol% ethanol-extracted fraction was confirmed to exhibit larger increase than the addition of the 50 µg/mL 60 vol% ethanol-extracted fraction (Figure 15).

## Claims

1. Kurosengoku *(Glycine max* cv. Kurosengoku) beans, crushed Kurosengoku beans, or an aqueous ethanol solution extract thereof for the use as immune balance modulator intended for activation of an antigen-presenting cell.

2. Kurosengoku *(Glycine max* cv. Kurosengoku) beans, crushed Kurosengoku beans, or an aqueous ethanol solution extract thereof for use according to claim 1, wherein the Kurosengoku beans are Kurosengoku beans swollen with water.

3. Kurosengoku *(Glycine max* cv. Kurosengoku) beans, crushed Kurosengoku beans, or an aqueous ethanol solution extract thereof for use according to claim 2, wherein the Kurosengoku beans swollen with water are Kurosengoku beans boiled in water or Kurosengoku beans steamed with water vapor.

4. Kurosengoku *(Glycine max* cv. Kurosengoku) beans, crushed Kurosengoku beans, or an aqueous ethanol solution extract thereof for use according to claim 1, wherein the crushed Kurosengoku beans are a crushed product of water-swollen Kurosengoku beans or a water-swollen product of crushed Kurosengoku beans.

5. Kurosengoku *(Glycine max* cv. Kurosengoku) beans, crushed Kurosengoku beans, or an aqueous ethanol solution extract thereof for use according to claim 4, wherein the crushed product of water-swollen Kurosengoku beans or the water-swollen product of crushed Kurosengoku beans are a crushed product of water-boiled Kurosengoku beans or a water-boiled product of crushed Kurosengoku beans, or a crushed product of water vapor-steamed Kurosengoku beans or a water vapor-steamed product of crushed Kurosengoku beans.

6. Kurosengoku *(Glycine max* cv. Kurosengoku) beans, crushed Kurosengoku beans, or an aqueous ethanol solution extract thereof for use according to claim 4 or 5, wherein the crushed Kurosengoku beans are crushed Kurosengoku beans that are collected into a supernatant fraction at 400xg and into a precipitate fraction at 18000xg in centrifugation with water as a mobile phase.

7. Kurosengoku *(Glycine max* cv. Kurosengoku) beans, crushed Kurosengoku beans, or an aqueous ethanol solution extract thereof for use according to any one of claims 2 to 6, wherein the Kurosengoku beans or the crushed Kurosengoku beans are further dehydrated.

8. Kurosengoku *(Glycine max* cv. Kurosengoku) beans, crushed Kurosengoku beans, or an aqueous ethanol solution extract thereof for use according to any one of claims 1 to 7, wherein the aqueous ethanol solution is a 30 vol% aqueous ethanol solution and/or a 60 vol% aqueous ethanol solution.

9. Kurosengoku *(Glycine max* cv. Kurosengoku) beans, crushed Kurosengoku beans, or an aqueous ethanol solution extract thereof for use according to any one of claims 1 to 7, wherein the aqueous ethanol solution extract is an extract that is collected into a supernatant fraction at 485xg in centrifugation with an aqueous ethanol solution as a mobile phase.

10. Kurosengoku *(Glycine max* cv. Kurosengoku) beans, crushed Kurosengoku beans, or an aqueous ethanol solution extract thereof for use according to any one of claims 1 to 7, wherein the Kurosengoku (*Glycine max* cv. Kurosengoku) beans, crushed Kurosengoku beans, or an aqueous ethanol solution extract thereof are used as food.

## Patentansprüche

1. Kurosengoku *(Glycine max* cv. Kurosengoku) Bohnen, zerstossene Kurosengoku Bohnen, oder ein Extrakt einer wässrigen Ethanollösung davon, für die Verwendung als Immun-Balance-Modulator vorgesehen für die Aktivierung einer antigenpräsentierenden Zelle.

2. Kurosengoku *(Glycine max* cv. Kurosengoku) Bohnen, zerstossene Kurosengoku Bohnen, oder ein Extrakt einer wässrigen Ethanollösung davon, für die Verwendung gemäss Anspruch 1, worin die Kurosengoku Bohnen mit Wasser gequollene Kurosengoku Bohnen sind.

3. Kurosengoku *(Glycine max* cv. Kurosengoku) Bohnen, zerstossene Kurosengoku Bohnen, oder ein Extrakt einer wässrigen Ethanollösung davon, für die Verwendung gemäss Anspruch 2, worin die mit Wasser gequollenen Kurosengoku Bohnen in Wasser gekochte oder mit Wasserdampf gedämpfte Kurosengoku Bohnen sind.

4. Kurosengoku *(Glycine max* cv. Kurosengoku) Bohnen, zerstossene Kurosengoku Bohnen, oder ein Extrakt einer wässrigen Ethanollösung davon, für die Verwendung gemäss Anspruch 1, worin die zerstossenen Kurosengoku Bohnen ein zerstossenes Produkt aus wassergequollenen Kurosengoku Bohnen oder ein wassergequollenes Produkt aus zerstossenen Kurosengoku Bohnen sind.

5. Kurosengoku (*Glycine max* cv. Kurosengoku) Bohnen, zerstossene Kurosengoku Bohnen, oder ein Extrakt einer wässrigen Ethanollösung davon, für die Verwendung gemäss Anspruch 4, worin das zerstossene Produkt aus wassergequollenen Kurosengoku Bohnen oder das wassergequollene Produkt aus zerstossenen Kurosengoku Bohnen ein zerstossenes Produkt aus wassergekochten Kurosengoku Bohnen oder ein wassergekochtes Produkt aus zerstossenen Kurosengoku Bohnen, oder ein zerstossenes Produkt aus wasserdampf-gedämpften Kurosengoku Bohnen oder ein wasserdampf-gedämpftes Produkt aus zerstossenen Kurosengoku Bohnen ist.

6. Kurosengoku (*Glycine max* cv. Kurosengoku) Bohnen, zerstossene Kurosengoku Bohnen, oder ein Extrakt einer wässrigen Ethanollösung davon, für die Verwendung gemäss Anspruch 4 oder 5, worin die zerstossenen Kurosengoku Bohnen zerstossene Kurosengoku Bohnen sind, die bei einer Zentrifugation mit Wasser als mobiler Phase in eine überstehende Fraktion bei 400xg und in eine Niederschlagsfraktion bei 18000xg hinein gesammelt werden.

7. Kurosengoku *(Glycine max* cv. Kurosengoku) Bohnen, zerstossene Kurosengoku Bohnen, oder ein Extrakt einer wässrigen Ethanollösung davon, für die Verwendung gemäss einem der Ansprüche 2 bis 6, worin die Kurosengoku Bohnen oder die zerstossenen Kurosengoku Bohnen weiter entwässert sind.

8. Kurosengoku (*Glycine max* cv. Kurosengoku) Bohnen, zerstossene Kurosengoku Bohnen, oder ein Extrakt einer wässrigen Ethanollösung davon, für die Verwendung gemäss einem der Ansprüche 1 bis 7, worin die wässrige Ethanollösung eine 30 vol%ige wässrige Ethanollösung und/oder eine 60 vol%ige wässrige Ethanollösung ist.

9. Kurosengoku (*Glycine max* cv. Kurosengoku) Bohnen, zerstossene Kurosengoku Bohnen, oder ein Extrakt einer wässrigen Ethanollösung davon, für die Verwendung gemäss einem der Ansprüche 1 bis 7, worin der Extrakt einer wässrigen Ethanollösung ein Extrakt ist, der bei einer Zentrifugation mit einer wässrigen Ethanollösung als mobiler Phase bei 485xg in eine überstehende Fraktion hinein gesammelt wird.

10. Kurosengoku (*Glycine max* cv. Kurosengoku) Bohnen, zerstossene Kurosengoku Bohnen, oder ein Extrakt einer wässrigen Ethanollösung davon, für die Verwendung gemäss einem der Ansprüche 1 bis 7, worin die Kurosengoku Bohnen, zerstossene Kurosengoku Bohnen, oder ein Extrakt einer wässrigen Ethanollösung davon als Lebensmittel verwendet werden.

## Revendications

1. Fèves de Kurosengoku (*Glycine max* cv. Kurosengoku), fèves de Kurosengoku broyées, ou un extrait de solution aqueuse d'éthanol de celles-ci pour leur utilisation en tant que modulateur d'équilibre immunitaire destiné à l'activation d'une cellule présentatrice d'antigène.

2. Fèves de Kurosengoku (*Glycine max* cv. Kurosengoku), fèves de Kurosengoku broyées, ou un extrait de solution aqueuse d'éthanol de celles-ci pour leur utilisation selon la revendication 1, dans lesquels les fèves de Kurosengoku sont des fèves de Kurosengoku gonflées avec de l'eau.

3. Fèves de Kurosengoku (*Glycine max* cv. Kurosengoku), fèves de Kurosengoku broyées, ou un extrait de solution aqueuse d'éthanol de celles-ci pour leur utilisation selon la revendication 2, dans lesquels les fèves de Kurosengoku gonflées avec de l'eau sont des fèves de Kurosengoku bouillies dans de l'eau ou des fèves de Kurosengoku cuites à la vapeur d'eau.

4. Fèves de Kurosengoku (*Glycine max* cv. Kurosengoku), fèves de Kurosengoku broyées, ou un extrait de solution aqueuse d'éthanol de celles-ci pour leur utilisation selon la revendication 1, dans lesquels les fèves de Kurosengoku broyées sont un produit broyé de fèves de Kurosengoku gonflées à l'eau ou un produit gonflé à l'eau de fèves de Kurosengoku broyées.

5. Fèves de Kurosengoku (*Glycine max* cv. Kurosengoku), fèves de Kurosengoku broyées, ou un extrait de solution aqueuse d'éthanol de celles-ci pour leur utilisation selon la revendication 4, dans lesquels le produit broyé de fèves de Kurosengoku gonflées à l'eau ou le produit gonflé à l'eau de fèves de Kurosengoku broyées sont un produit broyé de fèves de Kurosengoku bouillies à l'eau ou un produit bouilli à l'eau de fèves de Kurosengoku broyées, ou un produit broyé de fèves de Kurosengoku cuites à la vapeur d'eau ou un produit cuit à la vapeur d'eau de fèves de Kurosengoku broyées.

6. Fèves de Kurosengoku (*Glycine max* cv. Kurosengoku), fèves de Kurosengoku broyées, ou un extrait de solution aqueuse d'éthanol de celles-ci pour leur utilisation selon la revendication 4 ou 5, dans lesquels les fèves de Kurosengoku broyées sont des fèves de Kurosengoku broyées qui sont collectées dans une fraction de surnageant à 400xg et dans une fraction précipitée à 18000xg en centrifugation avec de l'eau en tant que phase mobile.

7. Fèves de Kurosengoku (*Glycine max* cv. Kurosengoku), fèves de Kurosengoku broyées, ou un extrait de solution aqueuse d'éthanol de celles-ci pour leur utilisation selon l'une quelconque des revendications 2 à 6, dans lesquels les fèves de Kurosengoku ou les fèves de Kurosengoku broyées sont ensuite déshydratées.

8. Fèves de Kurosengoku (*Glycine max* cv. Kurosengoku), fèves de Kurosengoku broyées, ou un extrait de solution aqueuse d'éthanol de celles-ci pour leur utilisation selon l'une quelconque des revendications 1 à 7, dans lesquels la solution aqueuse d'éthanol est une solution aqueuse d'éthanol à 30 % en volume et/ou une solution aqueuse d'éthanol à 60 % en volume.

9. Fèves de Kurosengoku (*Glycine max* cv. Kurosengoku), fèves de Kurosengoku broyées, ou un extrait de solution aqueuse d'éthanol de celles-ci pour leur utilisation selon l'une quelconque des revendications 1 à 7, dans lesquels l'extrait de solution aqueuse d'éthanol est un extrait qui est collecté dans une fraction de surnageant à 485xg en centrifugation avec une solution aqueuse d'éthanol en tant que phase mobile.

10. Fèves de Kurosengoku (*Glycine max* cv. Kurosengoku), fèves de Kurosengoku broyées, ou un extrait de solution aqueuse d'éthanol de celles-ci pour leur utilisation selon l'une quelconque des revendications 1 à 7, dans lesquels les fèves de Kurosengoku (*Glycine max* cv. Kurosengoku), les fèves de Kurosengoku broyées, ou un extrait de solution aqueuse d'éthanol de celles-ci sont utilisées comme aliment.
